# EUROPÄISCHE PATENTSCHRIFT

(11) **EP 0 502 820 B1**
(45) Veröffentlichungstag und Bekanntmachung des Hinweises auf die Patenterteilung: **21.09.1994**
(21) Anmeldenummer: 92810135.1
(22) Anmeldetag: 25.02.1992
(51) Int. Cl.: C07C 309/51, C07D 295/22

(54) **Neue Semicarbazide und ihre Verwendung zur Stabilisierung von Polyamid-Fasermaterialien und deren Färbungen**
New semicarbazides and their use for the stabilization of polyamide fibres and their colours
Nouveaux semi-carbazides et leur utilisation pour la stabilisation des fibres polyamides et leurs couleurs

(30) Priorität: 04.03.1991 CH 637/91
(43) Veröffentlichungstag der Anmeldung: 09.09.1992
(73) Patentinhaber: CIBA-GEIGY AG, 4002 Basel (CH)
(72) Erfinder: Kaschig, Jürgen, Dr., W-7800 Freiburg (DE); Reinert, Gerhard, Dr., W-4123 Allschwil (CH); Metzger, Georges, F-68480 Moernach (FR)

(56) Entgegenhaltungen:
- CHEMICAL ABSTRACTS, vol. 102, no. 20, 20. Mai 1985, Columbus, Ohio, US; abstract no. 168277U, MEISEI CHEMICAL WORKS: 'Improvement of heat resistance of dyed polyamide fibers' Seite 83 ;Spalte 2 ;
- CHEMICAL ABSTRACTS, vol. 103, no. 12, 23. September 1985, Columbus, Ohio, US; abstract no. 89053K, NIKKA CHEMICAL INDUSTRY: 'Finishing fiber products' Seite 64 ;Spalte 1 ;

## Beschreibung

Die vorliegende Erfindung betrifft neue, wasserlösliche Semicarbazide, ein Verfahren zur Herstellung dieser Verbindungen sowie ihre Verwendung zur photochemischen und thermischen Stabilisierung von Polyamidfasern und deren Färbungen.

Wasserlösliche Semicarbazide, die in der Lage sind, Fasern und deren Färbungen zu stabilisieren, sind bekannt. Diese Verbindungen haben jedoch den Nachteil, dass sie keine Affinitäten zu Polyamidfasern haben und somit unzureichende Wasser-, Wasch-, Schampoo- und Chemisch-Reinigungsechtheiten aufweisen. Ferner sind die anwendungstechnischen Möglichkeiten dieser Verbindungen auf Sprüh- oder Foulardierapplikation begrenzt.

Die erfindungsgemässen Semicarbazide hingegen sind wasserlöslich und gleichzeitig faseraffin und können in allen üblichen Färbe- und Nachbehandlungsverfahren eingesetzt werden, wobei mit diesen Verbindungen gute Nassechtheiten erzielt werden.

Gegenstand der vorliegenden Erfindung sind Semicarbazide der allgemeinen Formel 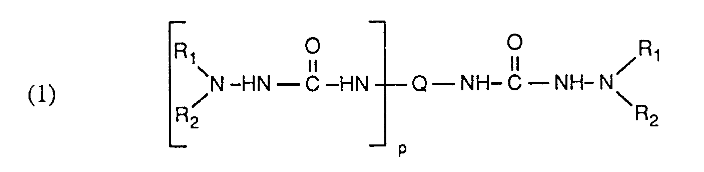
worin
R₁ und R₂, unabhängig voneinander, Wasserstoff, C₁-C₅alkyl, C₂-C₅alkenyl, C₁-C₅alkoxy oder Phenyl bedeuten oder R₁ und R₂ zusammen mit dem Stickstoffatom, an das sie gebunden sind, einen Morpholin- oder Piperazinrest bilden,
p 0 oder 1 ist,
wenn p=1 ist, Q als einen Rest der Formel 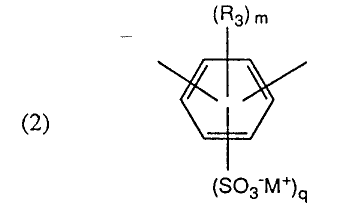
bedeutet, worin
R₃ Wasserstoff, C₁-C₅alkyl oder Halogen,
m 0,1, 2 oder 3,
q=1
oder, wenn p=0 ist,
Q einen Rest der Formel 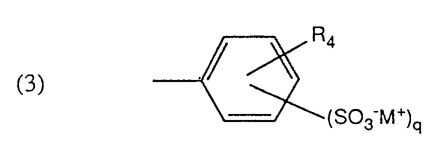
bedeutet, worin
R₄ Wasserstoff C₁-C₅alkyl, Halogen oder einen Rest der Formel 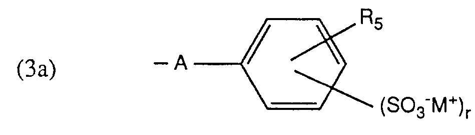
bedeutet, worin R₅ für Wasserstoff, C₁-C₅alkyl oder Halogen,
M Wasserstoff oder Alkali und
A -NH-, -O- oder -SO₂- bedeuten und q und r 0 oder 1, aber nicht gleichzeitig 0 sind.

Bei der Definition der Reste R₁, R₂, R₃ und R₅ stellen C₁-C₅alkyl und C₁-C₅alkoxy solche Gruppen oder Gruppenbestandteile dar, die 1 bis 5, insbesondere 1 bis 3 Kohlenstoffatome aufweisen. Beispiele für derartige Gruppen sind: Methyl, Ethyl, n-Propyl, Isopropyl, n-Butyl, sek.Butyl, tert.Butyl, Amyl oder Isoamyl bzw. Methoxy, Ethoxy, Isopropoxy, Isobutoxy, tert.-Butoxy oder tert.-Amyloxy.

C₂-C₅alkenyl bedeutet beispielsweise Vinyl, Propenyl, Butenyl oder vorzugsweise Allyl.

Halogen bedeutet Fluor, Brom oder Chlor. Chlor ist bevorzugt.

Als Beispiele für Alkalimetalle seien Lithium, Natrium oder Kalium genannt. Bevorzugt ist Natrium.

Phenyl kann durch eine oder mehrere (SO₃⁻M⁺)-Gruppen und C₁-C₅alkoxy durch C₁-C₅alkoxy substituiert sein.

Morpholino und Piperazinyl können durch einen oder mehrere C₁-C₃alkylreste substituiert sein.

Vorzugsweise kommen Verbindungen in Betracht, bei denen R₁ und R₂ in Formel 1 C₁-C₅alkyl bedeutet oder zusammen mit dem Stickstoffatom, an das sie gebunden sind, einen Morpholinring bilden,
Q als der Formel 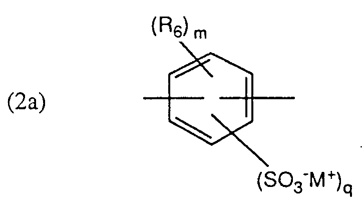
entspricht,
worin
R₆ C₁-C₅alkyl ist und
M, m und q die oben angegebene Bedeutung haben.

Bedeutet Q einen zweiwertigen Rest, sind besonders solche Verbindungen bevorzugt, bei denen R₁ und R₂ C₁-C₅alkyl und Q einen Rest der Formel 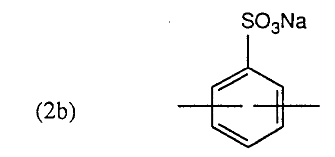
bedeutet.

Bedeutet Q einen einwertigen Rest, sind solche Verbindungen von Interesse, bei denen Q einen Rest der Formel 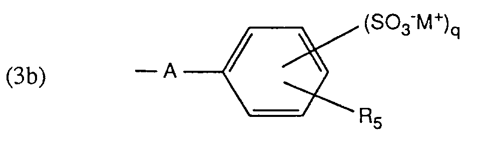
bedeutet, worin
R₅ C₁-C₅alkyl oder Halogen,
M Wasserstoff oder Alkali und
A -SO₂- oder -O- bedeuten.

Von ganz besonderem Interesse sind Semicarbazide, bei denen in Formel (1) R₁ und R₂ C₁-C₅alkyl und Q als einwertiger Rest der Formel 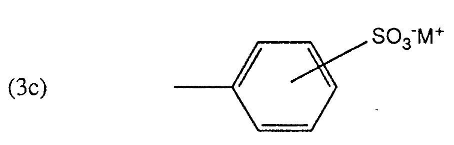
entspricht, worin M Wasserstoff oder Natrium bedeutet.

Die Herstellung der neuen Verbindungen erfolgt nach an sich bekannten Methoden, wie sie beispielsweise in Bull. Soc. Chim. France, S. D 12ff (1954) beschrieben sind.

Die erfindungsgemässen Semicarbazide werden hergestellt, indem man 1 Mol 1,1'-Carbonyldiimidazol mit einem Mol eines Hydrazins der Formel 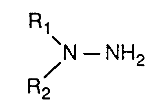
in wasserfreiem Medium in Anwesenheit eines polaren, aprotischen, mit Wasser mischbaren Lösungsmittels bei einer Temperatur von -20 bis +20°C, vorzugsweise -10 bis 0°C, und anschliessend mit einem Mol der Verbindung der Formel Q-NH₂ oder 0,5 Mol der Verbindung der Formel NH₂-Q-NH₂ in einem polaren, mit Wasser mischbaren Lösungsmittel oder mit Gemischen dieser Lösungsmittel mit Wasser bei einer Temperatur zwischen - 10 und 80°C, vorzugsweise 0 bis 30°C, nach dem Schema 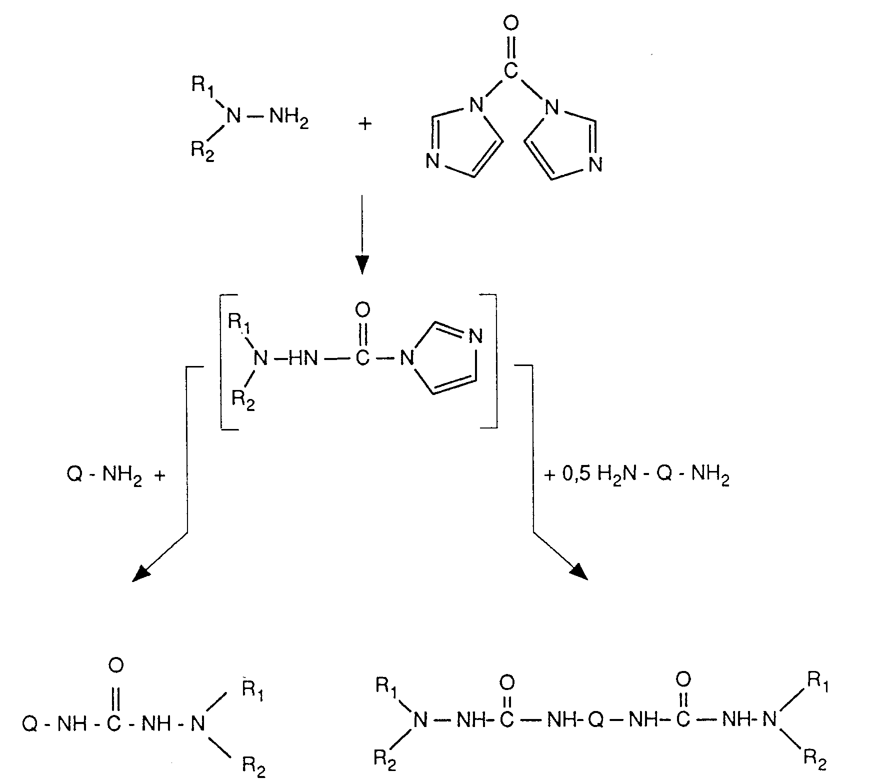
umsetzt. R₁ bis R₄, M, Q, m und q haben dabei die in den Formeln (1), (2), (3) und (3a) angegebene Bedeutung.

In der zweiten Stufe kommen als polare Lösungsmittel, die gegebenenfalls als Gemisch zusammen mit Wasser eingesetzt werden, wasserlösliche, organische Lösungsmittel in Betracht.

Beispiele dieser Lösungsmittel sind aliphatische C₁-C₄-Alkohole wie Methanol, Ethanol oder die Propanole; Alkylenglykole wie Ethylenglykol oder Propylenglykol; Monoalkylether von Glykolen wie Ethylenglykolmonomethyl-, -ethyl- oder -butylether und Diethylenglykolmonomethyl-oder ethylether; Ether und Acetale wie Diisopropylether, Diphenyloxid, Dioxan, Tetrahydrofuran, ferner Tetrahydrofurfurylalkohol, Pyridin, Acetonitril, γ-Butyrolacton, N,N-Dimethylformamid, N,N-Dimethylacetamid, Tetramethylharnstoff und Tetramethylensulfon. Bevorzugt sind Tetrahydrofuran oder N,N-Dimethylformamid, bzw. ihre Gemische mit Wasser.

Eine alternative Herstellungsmöglichkeit der neuen Verbindungen der Formel 1, worin Q so definiert ist, dass R₄ Wasserstoff, Halogen oder C₁-C₅alkyl bedeutet, besteht darin, dass man ein Mol Hydrazin der Formel 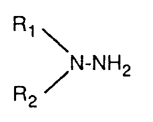
mit einem Mol eines Isocyanates der Formel Q'-NCO oder mit 0,5 Mol eines Diisocyanates der Formel OCN-Q''-NCO, worin R₁ und R₂ die in Formel 1 angegebene Bedeutung haben und Q' den Rest 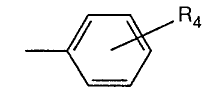
oder den Rest 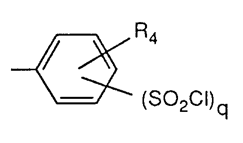
und Q'' den Rest 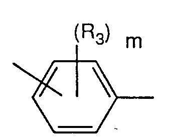
oder den Rest 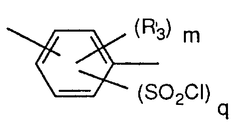
bedeuten, umsetzt und die erhaltenen Zwischenprodukte, wenn Q'den Rest 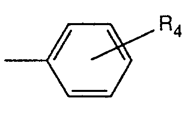
und Q'' den Rest 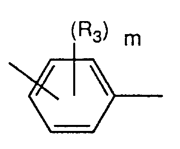
bedeuten, vorzugsweise mit Chlorsulfonsäure zu den Endprodukten sulfiert, und wenn Q' den Rest 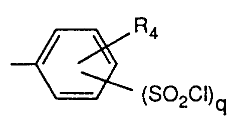
und Q'' den Rest 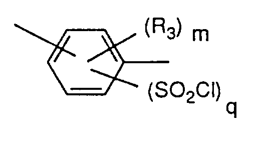
bedeuten, zu den Endprodukten hydrolisiert. R₃, R₄, m und q haben dabei die oben angegebene Bedeutung. Die Umsetzung erfolgt nach folgendem Schema: 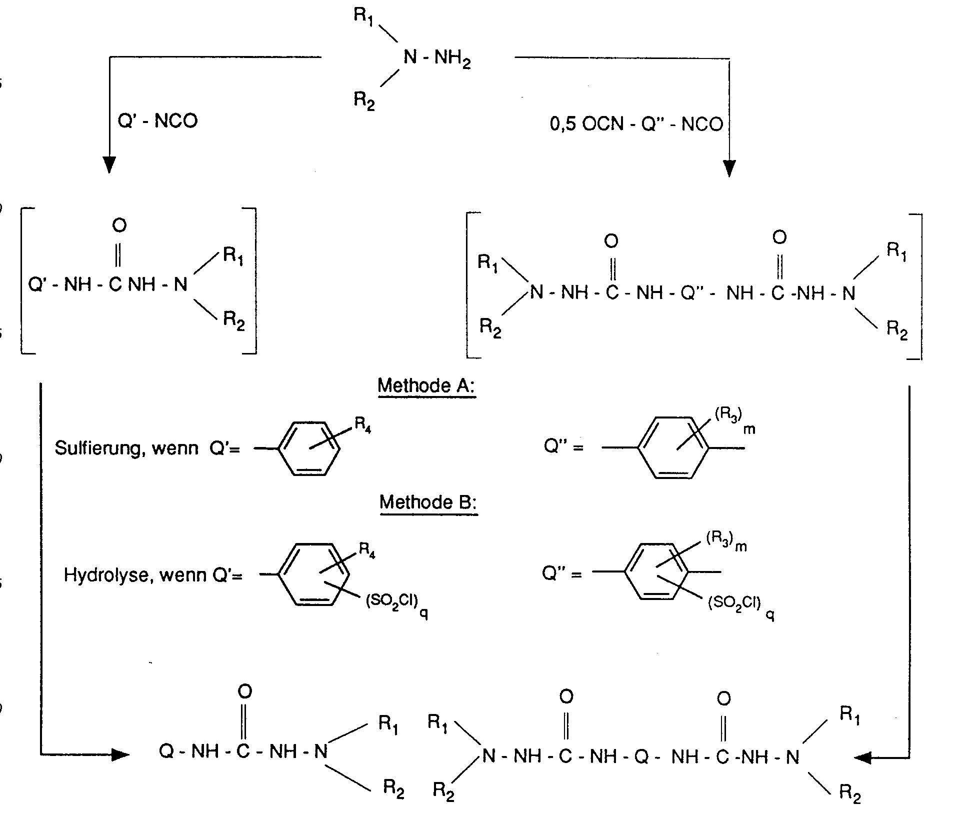
Die Zwischenprodukte werden nach den üblichen Verfahren hergestellt, vorzugsweise durch Addition von Hydrazinen an Isocyanate. Als Lösungsmittel kommen in Betracht: Carbonsäureester, z.B. Essigsäureester, halogenierte Kohlenwasserstoffe wie z.B. Tetrachlorkohlenstoff. Bevorzugtes Lösungsmittel ist Essigsäureethylester.

Die Verbindungen der Formel (1) werden erfindungsgemäss aus wässrigem Bad appliziert. Sie sind wasserlöslich, eenn sie in erfindungsgemässen, wässrigen Systemen appliziert werden. Die Verwendung von Dispergatoren ist nicht erforderlich. Die Menge der Zugesetzten Verbindungen hängt vom Substrat und der gewünschten Stabilisierung ab.

Im allgemeinen setzt man 0,01 bis 10 Gew.%, bevorzugt 0,1 bis 5 Gew.%, bezogen auf das Substrat, zu.

Die Applikation der erfindungsgemässen Verbindung kann vor, oder nach dem Färben, oder vorzugsweise während des Färbens, nach einem Ausziehverfahren bei Flottenverhältnissen von 1:5 bis 1:500, vorzugsweise 1:10 bis 1:50 erfolgen. Zweckmässigerweise werden die Verbindungen dem Färbebad zugesetzt.

Die erfindungsgemässen Verbindungen können aber auch kontinuierlich mittels Niedrigauftragssystemen oder Heissapplikatonssystemen appliziert werden.

Beim Kontinue-Verfahren beträgt der Flottenauftrag zweckmässig 30-400 Gew.-%, vorzugsweise 75-250 Gew.-%. Zur Fixierung der applizierten Farbstoffe und der bekannten und neuen Verbindungen wird das Fasermaterial einer Hitzebehandlung unterworfen. Der Fixierprozess kann auch nach der Kaltverweilmethode erfolgen.

Die Hitzebehandlung erfolgt vorzugsweise durch ein Dämpfverfahren unter Behandlung in einem Dämpfer mit gegebenenfalls überhitztem Dampf bei einer Temperatur von 98 bis 105°C während z.B. 1 bis 7, vorzugsweise 1 bis 5 Minuten. Die Fixierung der Farbstoffe und der Verbindungen der Formel (1) gemäss dem Kaltverweilverfahren kann durch Lagerung der imprägnierten und vorzugsweise aufgerollten Ware bei Raumtemperatur (15 bis 30°C), z.B. während 3 bis 24 Stunden erfolgen, wobei die Kaltverweilzeit bekanntlich von der Art des applizierten Farbstoffes abhängig ist.

Nach Beendigung des Färbeprozesses bzw. der Fixierung werden die hergestellten Färbungen auf übliche Weise gespült und getrocknet.

Die erfindungsgemässen Semicarbazide finden Verwendung zur photochemischen und thermischen Stabilisierung von Polyamidfasermaterialien und deren Färbungen. In ihrer Anwendung zeichnen sie sich durch eine gute Faseraffinität aus und verleihen den mit diesen Verbindungen behandelten Fasermaterialien eine gute photochemische Stabilität.

Unter Polyamidfasermateriäl wird dabei synthetisches Polyamid, wie z.B. Polyamid 6, Polyamid 66 oder auch Polyamid 12, verstanden. Neben den reinen Polyamidfasern kommen vor allem Fasermischungen aus Polyurethan und Polyamid in Betracht, so z.B. Trikotmaterial aus Polyamid/Polyurethan im Mischungsverhältnis 70:30. Ebenso kommen Fasermischungen aus Polypropylen und Polyamid in Frage. Grundsätzlich kann das reine oder gemischte Polyamidmaterial in verschiedenen Verarbeitungsformen vorliegen, wie z.B. als Faser, Garn, Gewebe, Gewirke oder Teppiche.

Vor allem Polyamidmaterial, auch in Mischungen mit Polyurethan oder Polypropylen, das Licht und Hitze ausgesetzt ist und z.B. als Autopolsterstoff, Teppich oder Badebekleidungsstoff vorliegt, eignet sich besonders für die Anwendung der erfindungsgemässen Verbindungen.

Die Färbung erfolgt in üblicher Weise, z.B. mit Metallkomplex-, Anthrachinon-oder Azofarbstoffen und Mischungen dieser Farbstoffe. Als Metallkomplexfarbstoffe werden die bekannten Typen, insbesondere die 1:2-Chrom- oder 1:2-Kobaltkomplexe von Mono-oder Disazo- oder Azomethinfarbstoffen eingesetzt, die in der Literatur in grosser Zahl beschrieben sind. Neben diesen kommen natürlich auch Farbstoffe aus anderen Farbstoffklassen in Frage, wie z.B. Dispersions- oder auch Reaktivfarbstoffe.

Die folgenden Herstellungsvorschriften der neuen Verbindungen und Anwendungsbeispiele dienen der Veranschaulichung der Erfindung. Teile bedeuten Gewichtsteile und Prozent Gewichtsprozente. Die Prozentangaben betreffend die Zusätze der einzelnen Färbe- bzw. Behandlungsbäder beziehen sich, wenn nicht anders angegeben, auf das Fasermaterial. Die Temperaturen sind in °C angegeben.

### Beispiel 1: 2,4-Bis(1,1-dimethylsemicarbazido-4)-benzolsulfonsäure Natriumsalz

Zu einer Lösung von 9,72 g (0,06 Mol) 1,1'-Carbonyldiimidazol in 130 ml Dimethylformamid wird bei -10° eine Lösung aus 3,6 g (0,06 Mol) 1,1-Dimethylhydrazin und 20 ml Dimethylformamid zugetropft. Nach 15 minütigem Rühren bei -10° werden 4,2 g (0,02 Mol) 1,3-Phenylendiamin-4-sulfonsäure Natriumsalz portionsweise zugegeben. Nach 16-stündigem Rühren bei Raumtemperatur wird bei 50° und leichtem Vakuum von ca. 0,13 Pa das Lösungsmittel weitgehend abdestilliert. Der Rückstand wird mit 300 ml Aceton aufgekocht und der verbleibende Niederschlag abgesaugt. Nach Trocknung bei 100° (ca. 0,13 Pa) werden 5,63 g der Verbindung der Formel 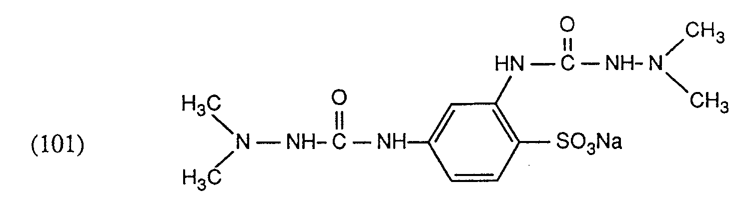
erhalten.

Ausbeute 76 %; Fₚ 230-235° (Zersetzung)

| Elementaranalyse: | | | | |
|---|---|---|---|---|
| Gefunden: | 37,78% C; | 4,87% H; | 21,74% N; | 8,04% S |
| Berechnet für C₁₂H₁₉N₆O₅SNa: | 37,69% C; | 5,01% H; | 21,98% N; | 8,39% S |

### Beispiel 2: 2,6-Bis(1,1-dimethylsemicarbazido-4)-mesitylen-4-sulfonsäure Natriumsalz

Entsprechend Beispiel 1 werden 2,7 g (0,045 Mol) 1,1-Dimethylhydrazin mit 7,29 g (0,045 Mol) 1,1'-Carbonyldiimidazol umgesetzt. Anschliessend werden 4,53 g (0,018 Mol) 2,6-Diamino-mesitylen-4-sulfonsäure Natriumsalz, gelöst in 25 ml Dimethylformamid, zugetropft. Nach 48-stündigem Rühren bei Raumtemperatur wird aufgearbeitet. Der nach Abdestillation von Dimethylformamid erhaltene Rückstand wird bei 55° mit 75 ml Isopropanol aufgenommen. Beim Abkühlen kristallisiert das Produkt aus. Nach Umkristallisieren aus Wasser werden 6,5 g der Verbindung der Formel 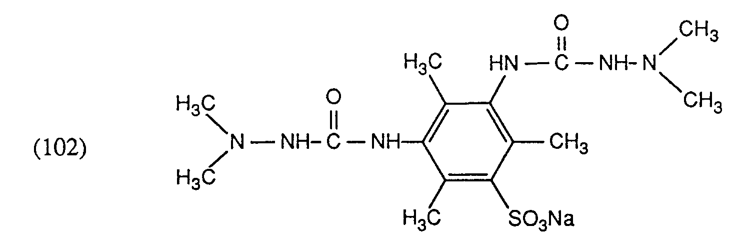
erhalten.

Ausbeute 85 %; Fₚ 250-260°

| | | | | |
|---|---|---|---|---|
| Gefunden: | 42,20% C; | 6,1% H; | 20,1% N; | 7,4% S |
| Berechnet für C₁₅H₂₅N₆O₅SNa: | 42,45% C; | 5,94% H; | 19,80% N; | 7,55% S |

### Beispiel 3: 4-(1,1-Dimethylsemicarbazido-4)-benzolsulfonsäure Natriumsalz

Entsprechend Beispiel 1 werden 1,28 g (0,0213 Mol) 1,1-Dimethylhydrazin mit 3,45 g (0,0213 Mol) 1,1'-Carbonyldiimidazol sowie 3,5 g (0,018 Mol) Sulfanilsäure Natriumsalz umgesetzt. Nach 24-stündigem Rühren bei Raumtemperatur und Abdestillieren des Lösungsmittels wird mit 100 ml Isopropanol verrührt und der ungelöste Rückstand abfiltriert. Dieser wird aus 90 ml Wasser umkristallisiert. Nach dem Trocknen bei 60° (0,13 Pa) werden 4,0 g der Verbindung der Formel 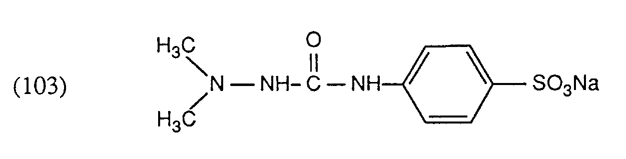
erhalten.

Ausbeute 79 %; Fₚ > 300°

| Elementaranalyse: | | | | |
|---|---|---|---|---|
| Gefunden: | 38,2% C; | 4,3% H; | 15,0% N; | 11,2% S |
| Berechnet für C₉H₁₂N₃O₄SNa: | 38,43% C; | 4,30% H; | 14,94% N; | 11,39% S |

### Beispiel 4: 3-(1,1-Dimethylsemicarbazido-4)-benzolsulfonsäure Natriumsalz

Zu einer Lösung von 7,29 g (0,045 Mol) 1,1'-Carbonyldiimidazol in 130 ml Tetrahydrofuran wird bei 0 bis 5° eine Lösung aus 2,7 g (0,045 Mol) 1,1-Dimethylhydrazin und 20 ml Tetrahydrofuran zugetropft. Nach 15-minütigem Rühren bei 0 bis 5° wird das Reaktionsgemisch zu einer Lösung aus 6,23 g (0,036 Mol) Metanilsäure, 36 ml 1n Natronlauge und 10 g Eis gegeben. Durch Zugabe von Wasser wird der entstandene Niederschlag in Lösung gebracht. Nach 4-stündigem Rühren bei Raumtemperatur wird zur Trockne eingedampft und der Rückstand mit 200 ml Isopropanol verrührt. Das verbleibende Pulver wird abgesaugt und getrocknet. Es werden 7,35 g der Verbindung der Formel 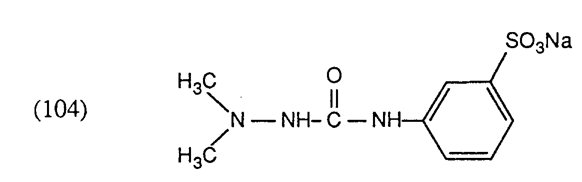
erhalten.

Ausbeute 72 %; Fₚ > 300°

| Elementaranalyse: | | | | |
|---|---|---|---|---|
| Gefunden: | 38,5% C; | 4,5% H; | 14,6% N; | 11,2% S |
| Berechnet für C₉H₁₂N₃O₄SNa: | 38,43% C; | 4,30% H; | 14,94% N; | 11,39% S |

### Beispiel 5: 3-(1,1-Dimethylsemicarbazido-4)-benzolsulfonsäure

In einer Lösung aus 0,8 g (2,84 mmol) der gemäss Beispiel 4 erhaltenen Verbindung und 2 ml Wasser werden 1,45 ml 2n Salzsäure gegeben. Nach 30-minütigem Rühren bei 0° wird der Niederschlag abgesaugt und nacheinander mit 2n Salzsäure und Ethanol gewaschen. Nach dem Trocknen im Exsikkator werden 0,41 g der Verbindung der Formel 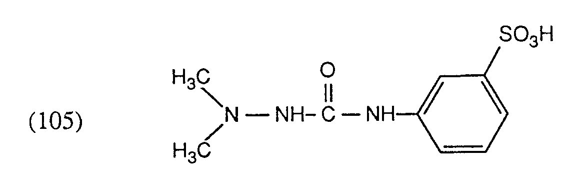
erhalten.

Ausbeute 56%; Fₚ 205-207°

| Elementaranalyse: | | | | |
|---|---|---|---|---|
| Gefunden: | 41,47% C; | 4,97% H; | 16,15% N; | 12,42% S |
| Berechnet für C₉H₁₃N₃O₄S: | 41,69% C; | 5,05% H; | 16,21% N; | 12,37% S |

### Beispiel 6: 2-(1,1-Dimethylsemicarbazido-4)-4,4'-dichlordiphenylether-2'-sulfonsäure

Entsprechend Beispiel 1 werden 2,7 g (0,045 Mol) 1,1-Dimethylhydrazin mit 7,29 g (0,045 Mol) 1,1'-Carbonyldiimidazol umgesetzt. Anschliessend werden 6,97 g (0,021 Mol) 2-Amino-4,4'-dichlordiphenylether-2'-sulfonsäure Natriumsalz, suspendiert in wenig Dimethylformamid, zugegeben. Nach 48-stündigem Rühren bei Raumtemperatur und anschliessendem Abdestillieren des Lösungsmittels werden 100 ml Wasser sowie 75 ml 2n Salzsäure zugegeben. Der Niederschlag wird abgesaugt und nacheinander mit 50 ml 2n Salzsäure und mit 400 ml Eiswasser gewaschen. Nach dem Trocknen im Exsikkator werden 7,81 g der Verbindung der Formel 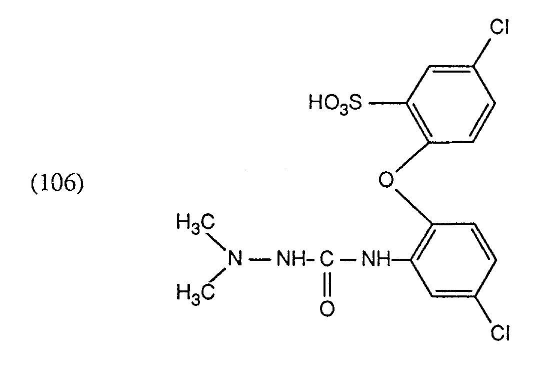
erhalten.

Ausbeute 89%; Fₚ 272-274°

| Elementaranalyse: | | | | |
|---|---|---|---|---|
| Gefunden: | 42,80% C; | 3,80% H; | 9.90% N; | 7,40% S |
| Berechnet für C₁₅H₁₅Cl₂N₃₆O₅S: | 42,76% C; | 3,82% H; | 9,97% N; | 7,61% S |

### Beispiel 7: 2-(1,1-Diaminosemicarbazido-4)-4'-methyldiphenylsulfon-4-sulfonsäure

Entsprechend Beispiel 1 werden 2,7 g (0,045 Mol) 1,1-Dimethylhydrazin mit 7,29 g (0,045 Mol) 1,1'-Carbonyldiimidazol umgesetzt. Anschliessend werden 3,95 g (0,0113 Mol) 2-Amino-4'-methyl-diphenylsulfon-4-sulfonsäure Natriumsalz, gelöst in 50 ml Dimethylformamid, zugegeben. Nach Beendigung der Reaktion wird entsprechend Beispiel 6 aufgearbeitet. Zur Reinigung wird das Rohprodakt 2 Stunden mit 150 ml Wasser verrührt und der Niederschlag abgesaugt und getrocknet. Man erhält 0,83 g der Verbindung der Formel 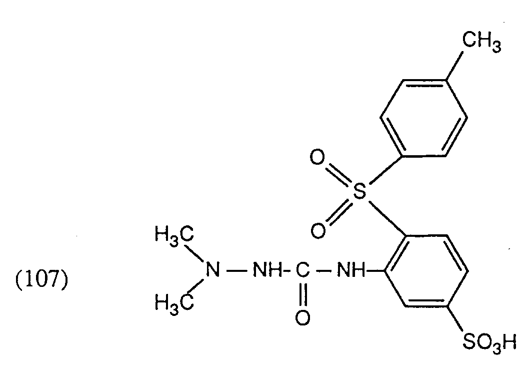
Ausbeute 18 %; Fₚ 260-262° (Zersetzung)

| Elementaranalyse: | | | |
|---|---|---|---|
| Gefunden: | 46,40% C; | 4,60% H; | 9,90% N; |
| Berechnet für C₁₆H₁₉N₃O₆S₂: | 46,48% C; | 4,63% H; | 10,16% N; |

### Beispiel 8: 2,4-Bis[1-(N-morpholino)-ureido-3]-benzolsulfonsäure Natriumsalz

Entsprechend Beispiel 1 werden 4,59 g (0,045 Mol) N-Aminomorpholin mit 7,29 g (0,045 Mol) 1,1'-Carbonyldiimidazol umgesetzt. Anschliessend werden 3,31 g (0,0157 Mol) 1,3-Phenylendiamin-4-sulfonsäure Natriumsalz, gelöst in Dimethylformamid, zugegeben. Nach 24 stündigem Rühren bei Raumtemperatur wird entsprechend Beispiel 2 aufgearbeitet. Nach dem Trocknen erhält man 5,77 g der Verbindung der Formel 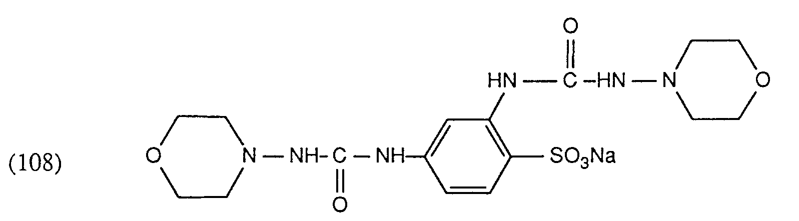
Sie enthält Kristall-Isopropanol. Ausbeute 76 %; Fₚ 256° (Zersetzung)

| Elementaranalyse: | | | | | |
|---|---|---|---|---|---|
| Gefunden: | 42,0% C; | 5,4% H; | 17,1% N; | 6,3% S; | 4,56% Na |
| Berechnet für C₁₆H₂₃N₆O₇SNa·1/3 C₃H₈O: | 42,0% C; | 5,3% H; | 17,3% N; | 6,6% S; | 4,76% Na |

### Beispiel 9: 4-(1,1-Dimethylsemicarbazido-4)benzolsulfonsäure

In einer Lösung aus 3 g (25,6 mmol) Chlorsulfonsäure und 30 ml Essigsäureethylester wird eine Lösung aus 2,24 g (12,5 mmol) 1,1-Dimethyl-4-phenylsemicarbazid und 30 ml Essigsäureethylester gegeben. Nach 10-minütigem Rühren bei 20° und 15-minütigem Rühren unter Rückfluss wird das Lösungsmittel unter leichtem Vakuum bei 40° abdestilliert. Der Rückstand wird mit 30 ml Eiswasser verrührt. Nach Absaugen des Niederschlages, Waschen mit 10 ml Eiswasser und Trocknen im Exsikkator werden 2,59 g Produkt der Formel 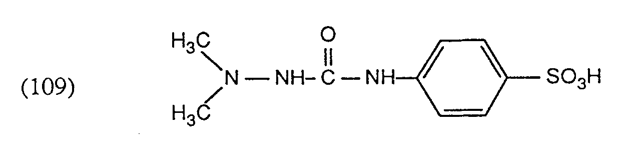
erhalten.

Ausbeute: 80%; Fₚ: 230-235° (Zersetzung)

| Elementaranalyse: | | | | |
|---|---|---|---|---|
| Gefunden: | 41,5% C; | 5,1% H; | 16,2% N; | 12,2% S |
| Berechnet für C₉H₁₃N₃O₄S: | 41,69% C; | 5,05% H; | 16,21% N; | 12,37% S |

### Applikationsbeispiele:

Beispiel 10: 2 Muster von je 10 g einer PA 6 - Maschenware werden in einem ®AHIBA-Färbeapparat bei einem Flottenverhältnis von 1:25 gefärbt. Beide Färbebäder enthalten folgende Zusätze:
0,5 g/l Mononatriumphosphat und
1,5 g/l Dinatriumphosphat, sowie folgende, in Wasser gelöste Farbstoffe der Formeln (I) und (II): 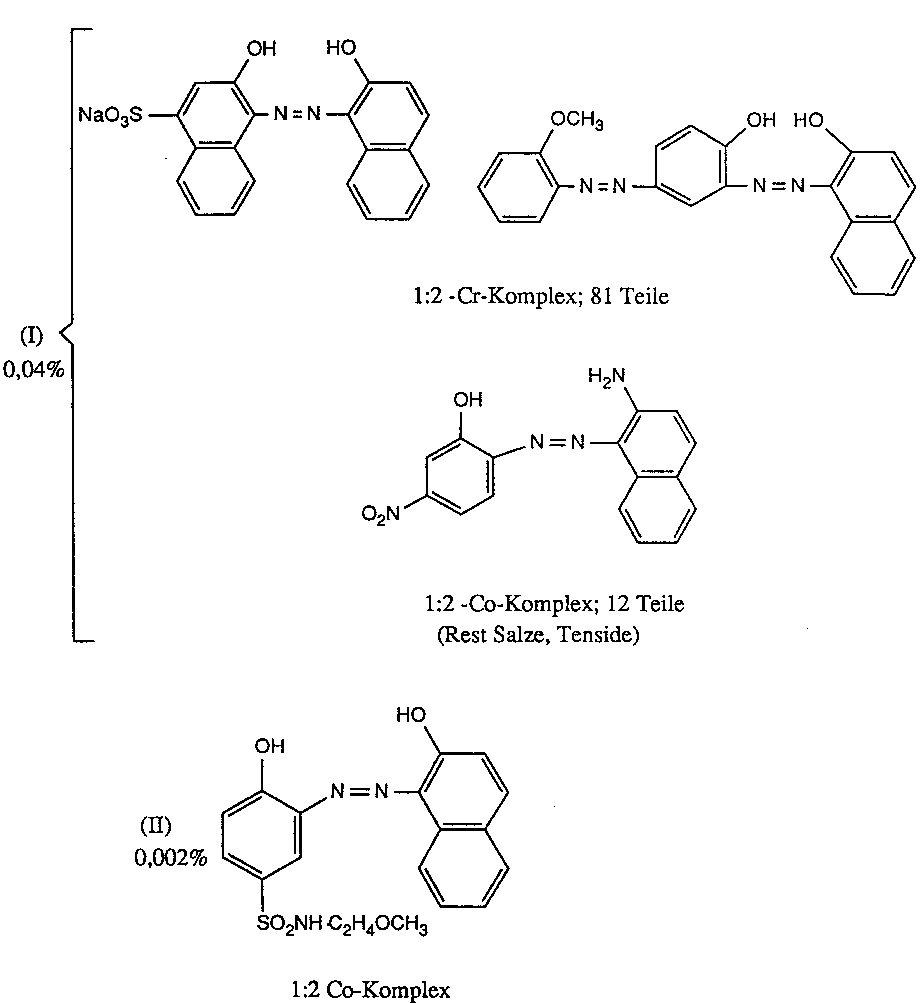
Während die Flotte 1 keine weiteren Zusätze enthält, wird der Flotte 2 noch 1% der Verbindung der Formel (101), in Wasser gelöst, zugesetzt.

In die so vorbereiteten Flotten geht man bei 40° ein, verweilt 10 Minuten bei dieser Temperatur und erhitzt mit 2°/Minute auf 95°. Nach einer Färbezeit von 20 Minuten bei 95° werden 2% Essigsäure (80%ig) zugesetzt und 25 Minuten weiterbehandelt. Danach kühlt man auf 60° ab, spült mit kaltem Wasser, zentrifugiert und trocknet bei 120° während 2 Minuten.

Die Färbungen werden auf ihre Lichtechtheiten nach SN-ISO 105-B02 (XENON) und DIN 75.202 (FAKRA) geprüft. Zur Ermittlung der photochemischen Stabilisierung werden die Färbungen in Mustern von 12x4,5 cm auf Karton aufgezogen, nach DIN 75.202 belichtet und nach SN 198.461 deren Reissfestigkeit und Dehnung ermittelt.

Beispiele 11 bis 13: Man verfährt wie in Beispiel 10 angegeben, mit dem Unterschied, dass jeweils folgende Verbindungen verwendet werden:
Beispiel 11: 1% der Verbindung der Formel (103)
Beispiel 12: 1% der Verbindung der Formel (104)
Beispiel 13: 1% der Verbindung der Formel (107)
Die Färbungen werden, wie in Beispiel 10 beschrieben, getestet. Die Ergebnisse der Beispiele 10 bis 13 sind aus Tabelle 1 zu entnehmen:

**Tabelle 1**

| Zusatz zum Färbebad | Lichtechtheit 2 FAKRA-Cyclen | Belichtung 216 | h FAKRA |
|---|---|---|---|
| | | Reissfestigkeit [%] | Dehnung [%] |
| Kein Zusatz | 1H | 15,7 | 33,1 |
| + Verbindung der Formel (101) | 3-4 | 63,6 | 70,0 |
| + Verbindung der Formel (103) | 3-4 | 67,7 | 72,0 |
| + Verbindung der Formel (104) | 2-3 | 62,8 | 70,2 |
| + Verbindung der Formel (107) | 2 | 56,1 | 61,7 |

Durch den Einsatz der Verbindungen der Formeln (101), (103), (104) und (107) werden eine deutliche Verbesserung der Photostabilität von Substrat und Färbung sowie eine wesentliche Verbesserung der Lichtechtheit der Färbungen erzielt.

## Patentansprüche

1. Semicarbazide der allgemeinen Formel 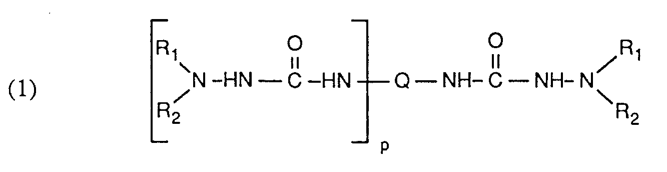 worin
R₁ und R₂, unabhängig voneinander, Wasserstoff, C₁-C₅alkyl, C₂-C₅alkenyl, C₁-C₅alkoxy oder Phenyl bedeuten oder
R₁ und R₂ zusammen mit dem Stickstoffatom, an das sie gebunden sind, einen Morpholin-oder Piperazinrest bilden,
p 0 oder 1 ist,
wenn p=1 ist, Q als zweiwertiger Rest einen Rest der Formel 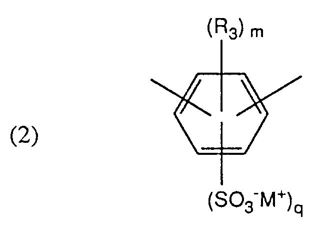 bedeutet, worin
R₃ Wasserstoff, C₁-C₅alkyl oder Halogen,
m 0,1, 2 oder 3,
q=1
oder, wenn p=0 ist,
Q einen Rest der Formel 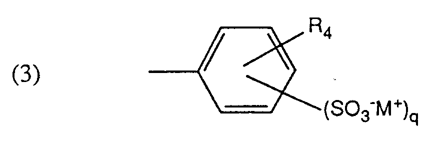 bedeutet, worin
R₄ Wasserstoff C₁-C₅alkyl, Halogen oder einen Rest der Formel 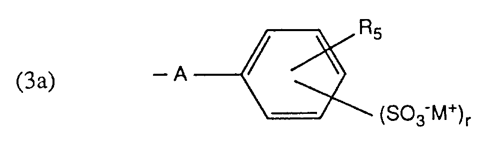 bedeutet, worin
R₅ für Wasserstoff, C₁-C₅alkyl oder Halogen,
M für Wasserstoff oder Alkali und
A für -NH-, -O- oder -SO₂- stehen und q und r 0 oder 1, aber nicht gleichzeitig 0 bedeuten.

2. Semicarbazide gemäss Anspruch 1, worin R₁ und R₂ in Formel 1 C₁-C₅alkyl bedeutet oder R₁ und R₂ zusammen mit dem Stickstoffatom, an das sie gebunden sind, einen Morpholinring bilden ,
Q als zweiwertiger Rest der Formel 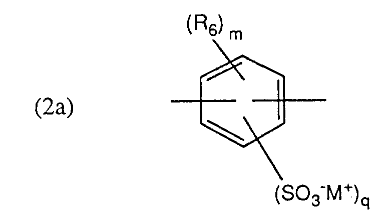 entspricht, worin
R₆ C₁-C₅alkyl ist und
M, m und q die in Anspruch 1 angegebene Bedeutung haben.

3. Semicarbazide gemäss Anspruch 1 oder 2, worin R₁ und R₂ C₁-C₅alkyl und Q einen zweiwertigen Rest der Formel 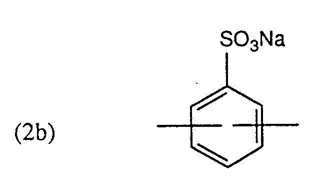 bedeutet.

4. Semicarbazide gemäss Anspruch 1, worin Q als einwertiger Rest einen Rest der Formel 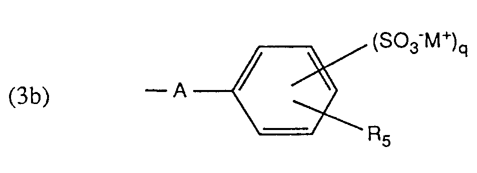 bedeutet, worin
R₅ C₁-C₅alkyl oder Halogen,
M Wasserstoff oder Alkali und
A-SO₂ oder -O- bedeuten.

5. Semicarbazide gemäss einem der Ansprüche 1 oder 4, worin in Formel (1) R₁ und R₂ C₁-C₅alkyl und Q als einwertiger Rest der Formel 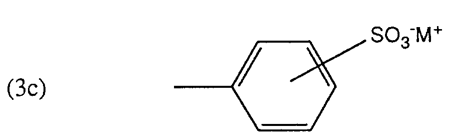 entspricht, worin M Wasserstoff oder Natrium bedeutet.

6. Verfahren zur Herstellung der Semicarbazide gemäss Anspruch 1, dadurch gekennzeichnet, dass man 1 Mol 1,1'-Carbonyldiimidazol mit einem Mol eines Hydrazins der Formel 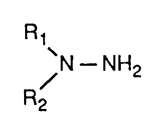 in wasserfreiem Medium in Anwesenheit eines polaren, mit Wasser mischbaren Lösungsmittels bei einer Temperatur von -20 bis +20°C und anschliessend mit einem Mol der Verbindung der Formel Q-NH₂ oder 0,5 Mol der Verbindung der Formel NH₂-Q-NH₂ in einem polaren, mit Wasser mischbaren Lösungsmittel oder mit Gemischen dieser Lösungsmittel mit Wasser bei einer Temperatur zwischen -10 und80°C nach dem Schema 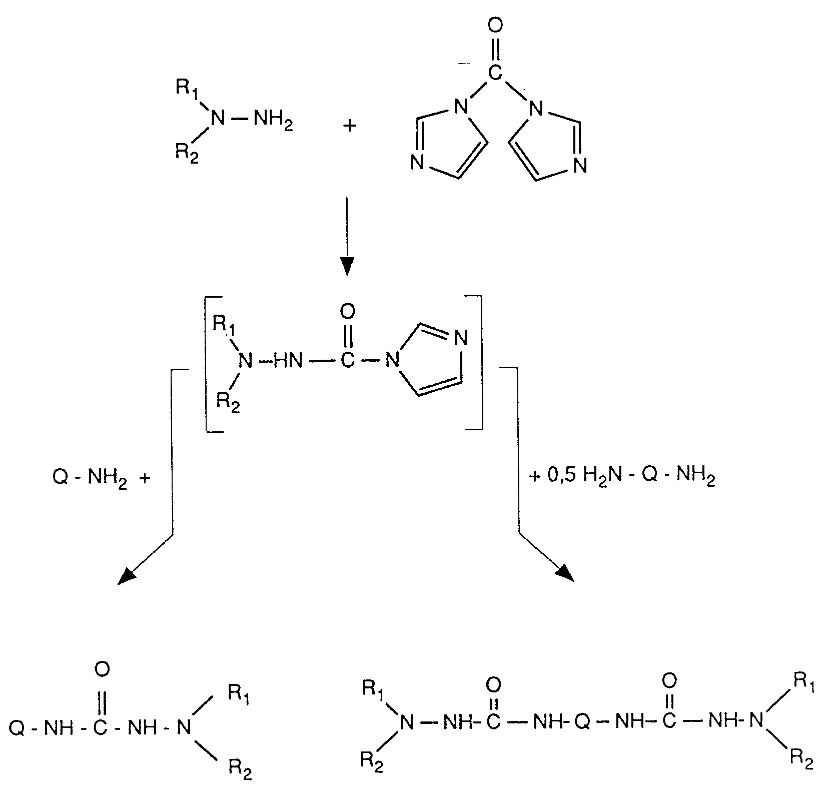 umsetzt, wobei
die Reste R₁ bis R₄, M, Q, m und q die in Anspruch 1 angegebene Bedeutung haben.

7. Verfahren zur Herstellung der Semicarbazide gemäss Anspruch 1, worin R₄ Wasserstoff, Halogen oder C₁-C₅alkyl bedeutet, dadurch gekennzeichnet, dass man ein Mol Hydrazin der Formel 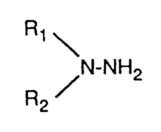 mit einem Mol eines Isocyanates der Formel Q'-NCO oder mit 0,5 Mol eines Diisocyanates der Formel OCN-Q''-NCO, worin R₁ und R₂ die in Formel 1 angegebene Bedeutung haben und Q' den Rest 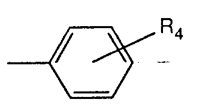 oder den Rest 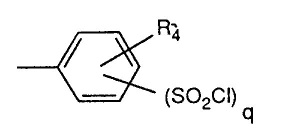 und Q'' den Rest 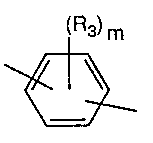 oder den Rest 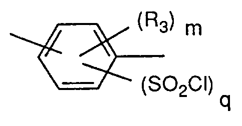 bedeuten, umsetzt und die erhaltenen Zwischenprodukte, wenn Q'den Rest 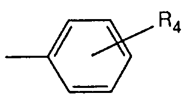 und Q'' den Rest 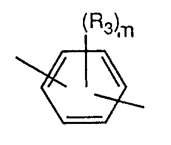 bedeuten, vorzugsweise mit Chlorsulfonsäure zu den Endprodukten sulfiert, und wenn Q' den Rest 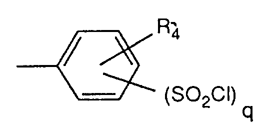 und Q'' den Rest 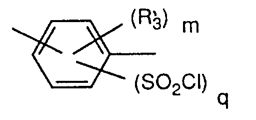 bedeuten, zu den Endprodukten hydrolisiert, worin R₃, R₄, m und q die in Anspruch 1 angegebene Bedeutung haben, entsprechend folgendem Schema:

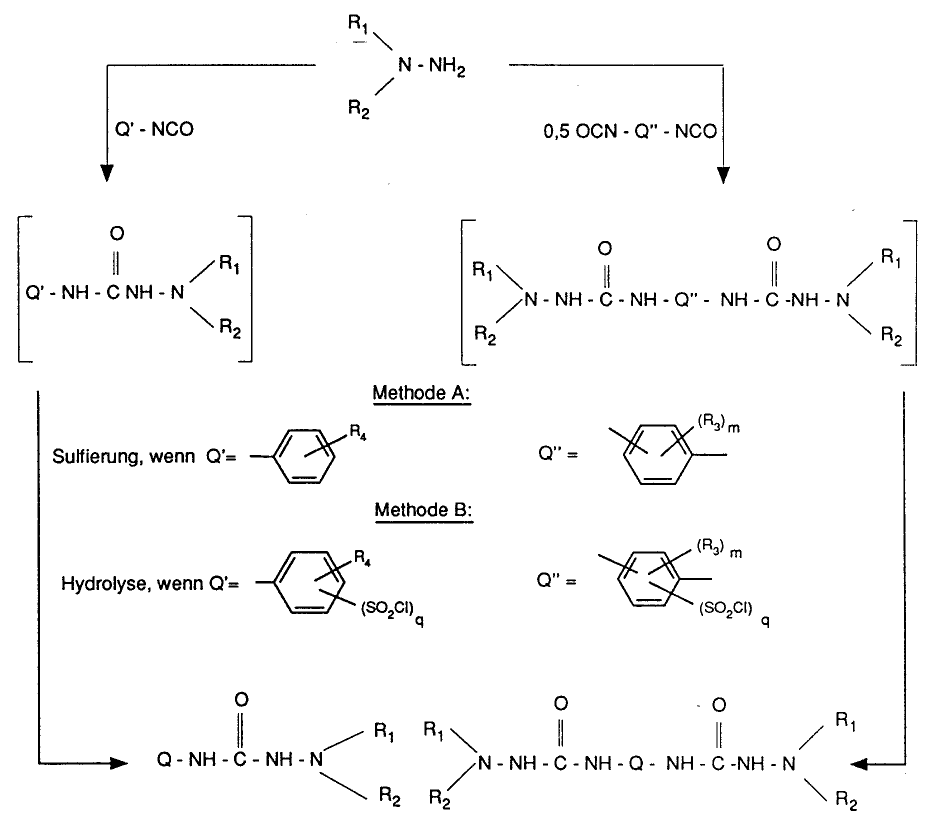

8. Verwendung der Semicarbazide gemäss einem der Ansprüche 1 bis 5 zur photochemischen und thermischen Stabilisierung von Polyamidfasermaterialien und deren Färbungen.

9. Das mit den Semicarbaziden gemäss einem der Ansprüche 1 bis 7 behandelte Fasermaterial.

## Claims

1. A semicarbazide of general formula 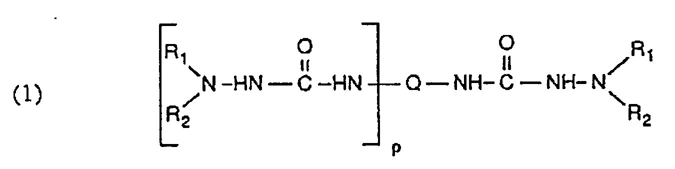 wherein
R₁ and R₂, each independently of the other, are hydrogen, C₁-C₅alkyl, C₂-C₅alkenyl, C₁-C₅alkoxy or phenyl, or R₁ and R₂, together with the nitrogen atom to which they are attached, are a morpholine or piperazine radical,
p is 0 or 1,
when p=1, Q is a divalent radical of formula 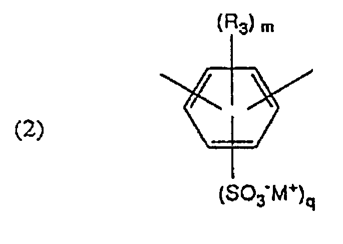 wherein
R₃ is hydrogen, C₁-C₅alkyl or halogen,
m is 0, 1, 2 or 3,
q=1
or, when p=0,
Q is a radical of formula 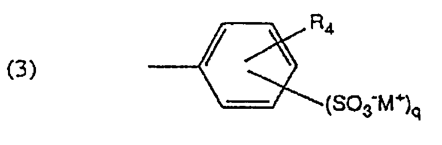 wherein
R₄ is hydrogen, C₁-C₅alkyl, halogen or a radical of formula 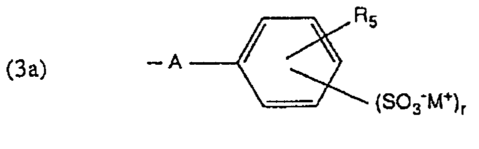 wherein R₅ is hydrogen, C₁-C₅alkyl or halogen,
M is hydrogen or alkali, and
A is -NH-, -O- or -SO₂-, and q and r are 0 or 1, but are not simultaneously 0.

2. A semicarbazide according to claim 1, wherein R₁ and R₂ in formula 1 are C₁-C₅alkyl or R₁ and R₂, together with the nitrogen atom to which they are attached, form a morpholine ring,
Q is a divalent radical of formula 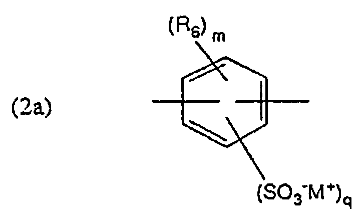 wherein
R₆ is C₁-C₅alkyl, and
M, m and q are as defined in claim 1.

3. A semicarbazide according to claim 1 or 2, wherein R₁ and R₂ are C₁-C₅alkyl and Q is a divalent radical of formula

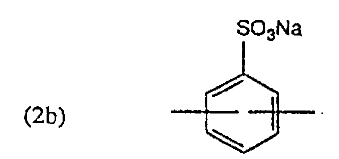

4. A semicarbazide according to claim 1, wherein Q is a monovalent radical of formula 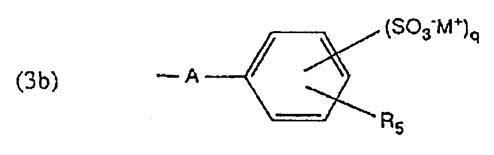 wherein
R₅ is C₁-C₅alkyl or halogen,
M is hydrogen or alkali, and
A is -SO₂ or -O-.

5. A semicarbazide according to either claim 1 or 4, wherein in formula (1) R₁ and R₂ are C₁-C₅alkyl and Q is a monovalent radical of formula 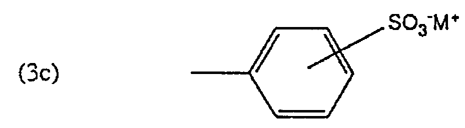 wherein M is hydrogen or sodium.

6. A process for the preparation of a semicarbazide according to claim 1, which comprises reacting 1 mol of 1,1'-carbonyldiimidazole with one mole of a hydrazine of formula 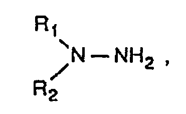 in anhydrous medium and in the presence of a polar, water-miscible solvent at a temperature from -20 to +20°C, and subsequently reacting the intermediate product with one mole of the compound of formula Q-NH₂ or with 0.5 mol of the compound of formula NH₂-Q-NH₂, in a polar, water-miscible solvent or with a mixture of said solvents with water, at a temperature between -10 and +80°C, according to the scheme 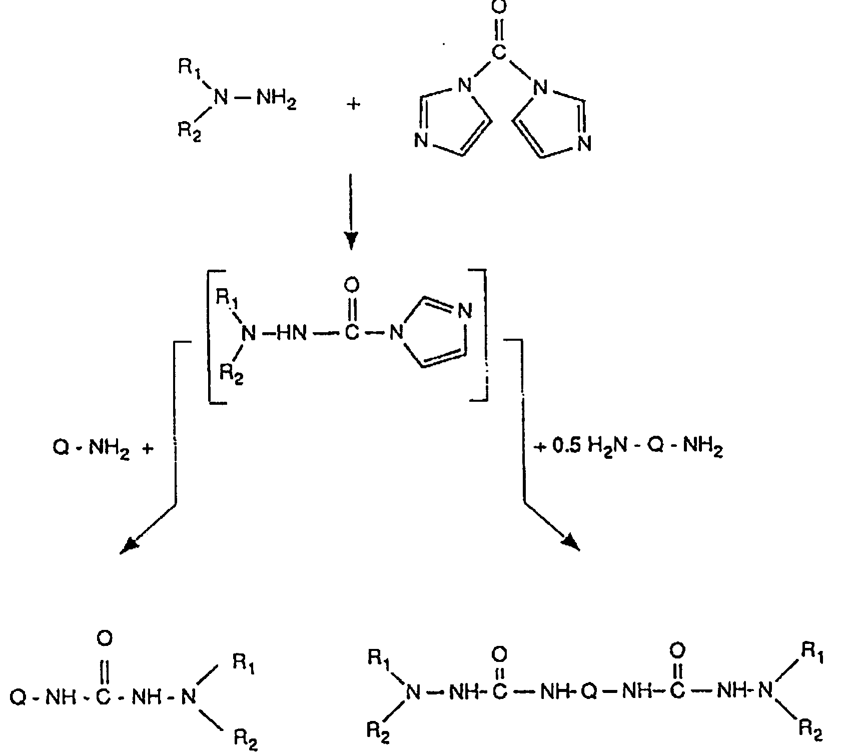 wherein the radicals R₁ to R₄, M, Q, m and q are as defined in claim 1.

7. A process for the preparation of a semicarbazide according to claim 1, wherein R₄ is hydrogen, halogen or C₁-C₅alkyl, which comprises reacting one mole of a hydrazine of formula 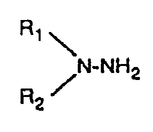 with one mole of an isocyanate of formula Q'-NCO or with 0.5 mol of a diisocyanate of formula OCN-Q''-NCO, wherein R₁ and R₂ are as defined in formula 1 and Q' is the radical 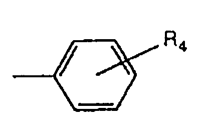 or the radical 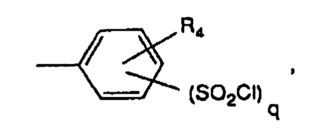 and Q'' is the radical 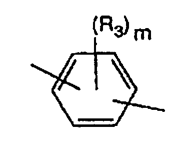 or the radical 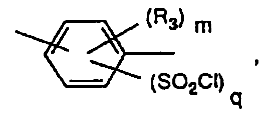 and, if Q' is the radical 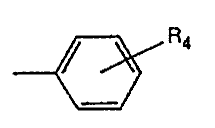 and Q'' is the radical 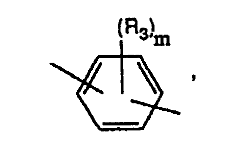 sulfonating the resultant intermediate product duct, preferably with chlorosulfonic acid, to give the final product, and, if Q' is the radical 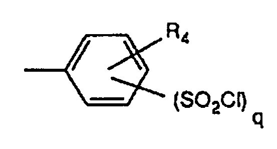 and Q'' is the radical 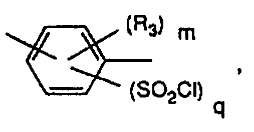 hydrolysing the intermediate product to give the final product, in which R₃, R₄, m and q are as defined in claim 1, in accordance with the following scheme:

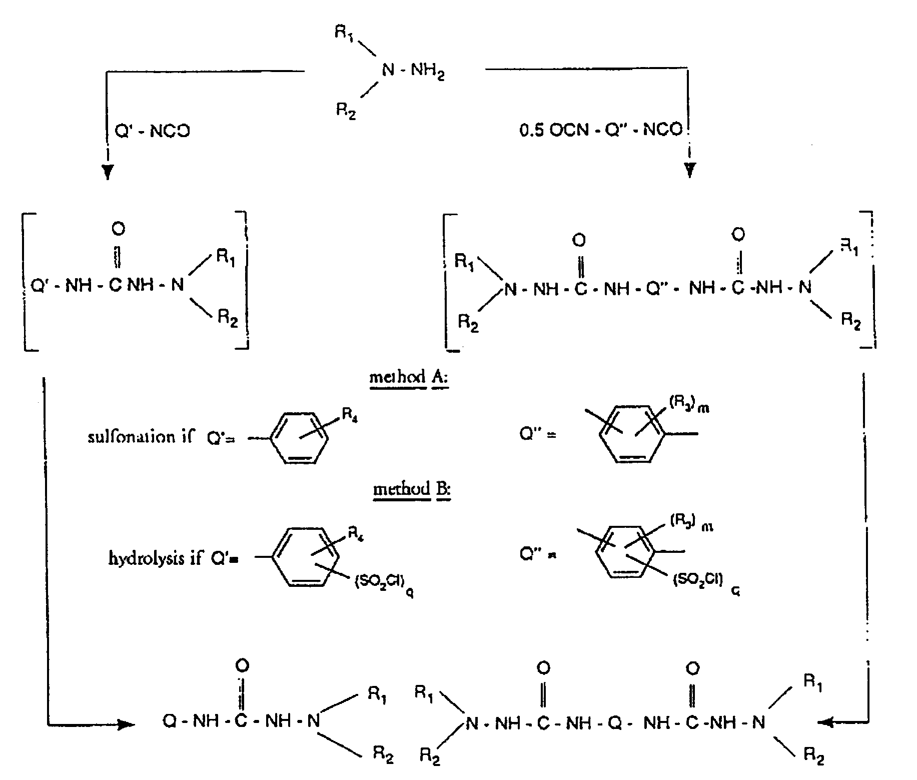

8. Use of a semicarbazide according to one of claims 1 to 5 for the photochemical and thermal stabilization of polyamide fibre materials and the dyeings produced thereon.

9. A fibre material treated with a semicarbazide according to one of claims 1 to 7.

## Revendications

1. Semi-carbazides de formule générale 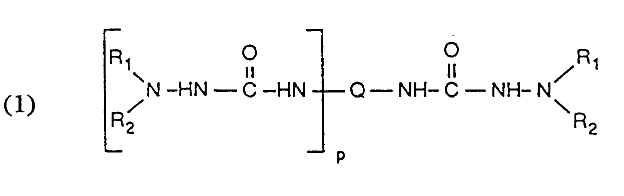 dans laquelle R₁ et R₂ représentent indépendamment l'un de l'autre, un atome d'hydrogène, un groupe alkyle en C₁-C₅, alcényle en C₂-C₅, alcoxy en C₁-C₅ ou phényle, ou R₁ et R₂ forment ensemble avec l'atome d'azote auquel ils sont liés un résidu de morpholine ou de pipérazine,
p vaut 0 ou 1,
lorsque p = 1, Q représente un résidu divalent de formule 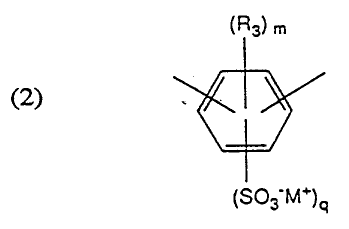 dans laquelle R₃ représente un atome d'hydrogène, un groupe alkyle en C₁-C₅ ou un atome d'halogène,
m vaut 0, 1, 2 ou 3,
q = 1
ou, lorsque p = 0, Q représente un résidu de formule 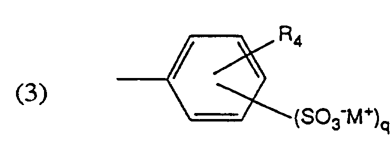 dans laquelle R₄ représente un atome d'hydrogène, d'halogène, un groupe alkyle en C₁-C₅ ou un résidu de formule 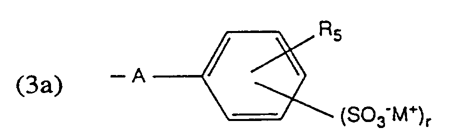 dans laquelle R₅ représente un atome d'hydrogène, d'halogène ou un groupe alkyle en C₁-C₅,
M représente un atome d'hydrogène ou un métal alcalin et A représente un groupe -NH-, -O- ou -SO₂- et q et r sont égaux à 0 ou 1 mais jamais en même temps égaux à 0.

2. Semi-carbazides conformes à la revendication 1, dans lesquels R₁ et R₂ dans la formule 1 sont des groupes alkyle en C₁-C₅ ou forment avec l'atome d'azote auquel ils sont liés un noyau morpholine,
Q correspond à un résidu bivalent de formule 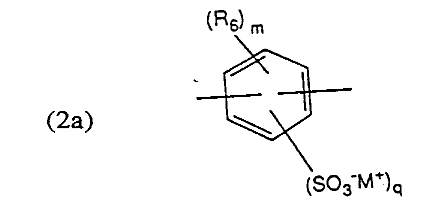 dans laquelle R₆ est un groupe alkyle en C₁-C₅, et
M, m et q ont la signification indiquée ci-dessus.

3. Semi-carbazides conformes à la revendication 1 ou 2, dans lesquels R₁ et R₂ représentent un groupe alkyle en C₁-C₅, et Q un résidu bivalent de formule

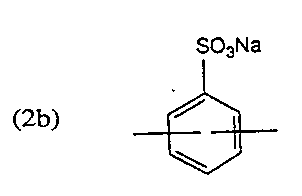

4. Semi-carbazides conformes à la revendication 1, dans lesquels Q, lorsqu'il s'agit d'un radical monovalent est un résidu de formule 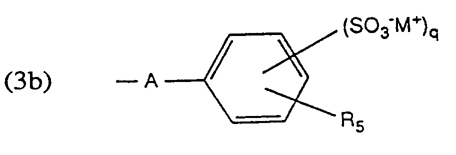 dans laquelle R₅ représente un groupe alkyle en C₁-C₅ ou un atome d'halogène, M un atome d'hydrogène ou un métal alcalin et A un groupe -SO₂- ou -O-.

5. Semi-carbazides conformes à une des revendications 1 ou 4, dans lesquels, dans la formule (1), R₁ et R₂ représentent un groupe alkyle en C₁-C₅ et Q correspond à un résidu monovalent de formule 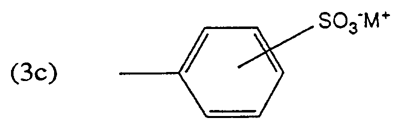 dans laquelle M est un atome d'hydrogène ou de sodium.

6. Procédé de préparation de semi-carbazides conforme à la revendication 1, caractérisé en ce que l'on fait réagir 1 mole de 1,1'-carbonyldiimidazole avec une mole d'une hydrazine de formule 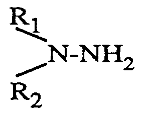 dans un milieu anhydre en présence d'un solvant polaire, miscible avec l'eau à une température de -20 à +20 °C, puis avec une mole d'un composé de formule Q-NH₂ ou 0,5 mole d'un composé de formule NH₂-Q-NH₂ dans un solvant polaire miscible avec l'eau ou dans des mélanges de ces solvants avec l'eau, à une température comprise entre -10 et 80 °C, selon le schéma réactionnel 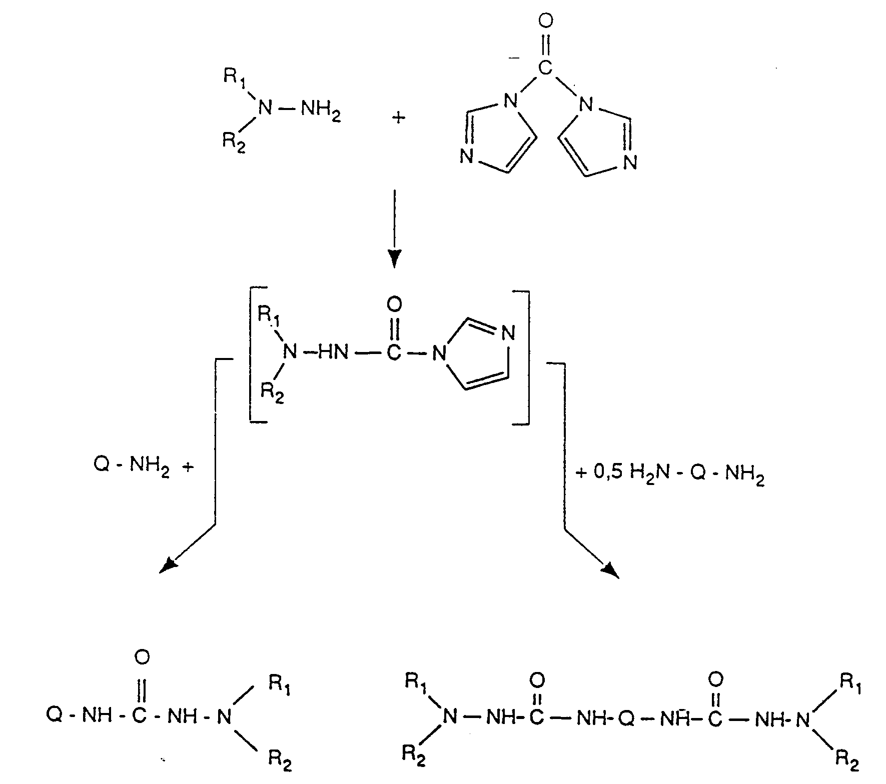 R₁ à R₄, M, Q, m et q ayant la signification indiquée dans la revendication 1.

7. Procédé de préparation des semi-carbazides conformes à la revendication 1, dans lequel R₄ représente un atome d'hydrogène, d'halogène ou un groupe alkyle en C₁-C₅, caractérisé en ce que l'on fait réagir une mole d'hydrazine de formule 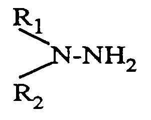 avec une mole d'un isocyanate de formule Q'-NCO ou avec 0,5 mole d'un diisocyanate de formule OCN-Q''-NCO, où R₁ et R₂ ont la signification indiquée dans la formule 1, Q' représente un résidu de formule 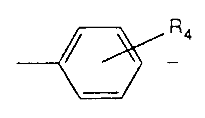 ou un résidu de formule 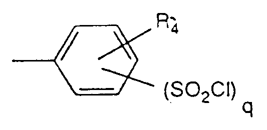 et Q'' un résidu de formule 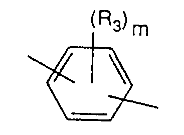 ou un résidu de formule 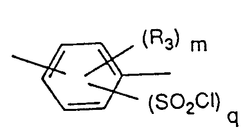 et que l'on sulfone les produits intermédiaires en pro duits finaux de préférence avec de l'acide chlorosulfonique, lorsque Q' est un résidu 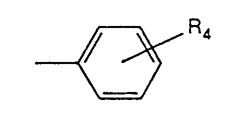 et Q'' un résidu 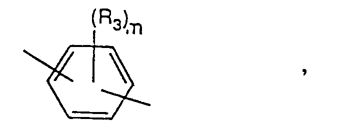 et que l'on hydrolyse les produits intermédiaires en produits finaux lorsque Q' est un résidu 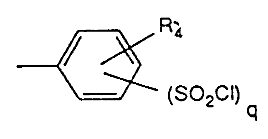 et Q'' un résidu 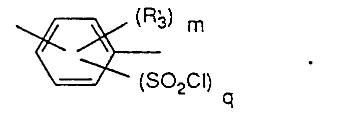 R₃, R₄, m et q ont alors les significations indiquées dans la revendication 1. La réaction se déroule selon le schéma réactionnel suivant :

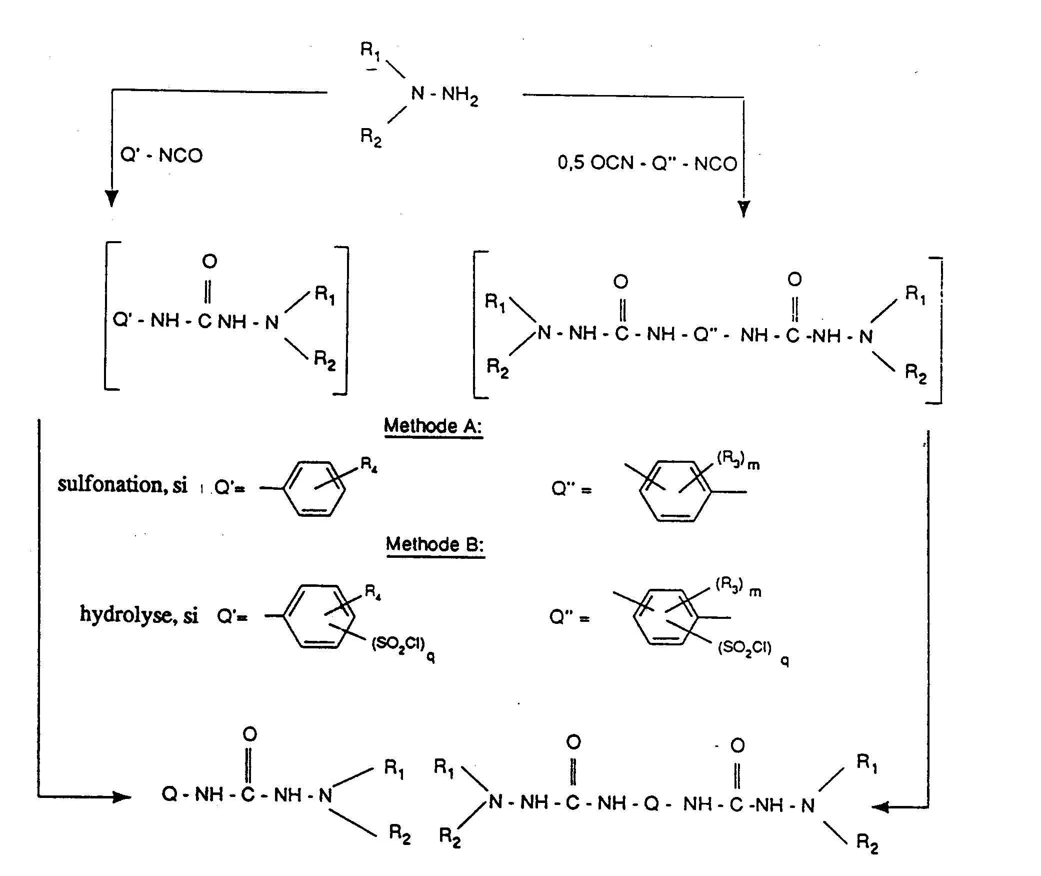

8. Utilisation des semi-carbazides conformes à une des revendications 1 à 5 pour la stabilisation photochimique et thermique de matériaux fibreux en polyamide et de leurs teintures.

9. Matériau fibreux traité par les semi-carbazides conformes à une des revendications 1 à 7.
